Europäisches Patentamt

European Patent Office

Office européen des brevets

(11) Veröffentlichungsnummer: **0 396 809 A1**

## (12) EUROPÄISCHE PATENTANMELDUNG

(21) Anmeldenummer: **89108584.7**

(22) Anmeldetag: **12.05.89**

(51) Int. Cl.5: **A61F 2/06**

(43) Veröffentlichungstag der Anmeldung:
**14.11.90 Patentblatt 90/46**

(84) Benannte Vertragsstaaten:
**AT BE CH DE ES FR GB GR IT LI LU NL SE**

(71) Anmelder: **A.J.N. COLLAGEN FORSCHUNGS-
UND VERTRIEBS GMBH
Hans-Urmiller-Ring 55
D-8190 Wolfratshausen(DE)**

(72) Erfinder: **Sedlarik K.M.Dr.med.
Postbus 30.001, Oostersingel 59
Zusterhuis RB 9700 Groningen ARG(NL)**

(54) **Künstliche kleinlumige Gefässprothese und Verfahren zu deren Herstellung.**

(57) Die Erfindung betrifft eine künstliche kleinlumige Gefäßprothese mit folgendem Aufbau von innen nach außen:

A. kleinlumiger mikroporöser Schlauch;

B. Mesh-graft aus autogenem Venenmaterial, das sich enganliegend an der äußeren Oberfläche von A. befindet, und

C. Netz aus einem resorbierbaren Material zum Fixieren von B. auf A.

Sie betrifft auch ein Verfahren zu deren Herstellung. Nach dem Implantieren einer solchen künstlichen Gefäßprothese bildet sich innerhalb sehr kurzer Zeit eine ausreichende Endothelialisierung im Inneren der Prothese.

EP 0 396 809 A1

Xerox Copy Centre

## Künstliche kleinlumige Gefäßprothese und Verfahren zu deren Herstellung

Die Erfindung betrifft kleinlumige künstliche Gefäßprothesen und ein Verfahren zu deren Herstellung. Sie befaßt sich insbesondere mit künstlichen Arterien.

Kleine Arterien, die aus verschiedenen Gründen beim Menschen ersetzt werden müssen, können nur aus körpereigenen Gefäßen (meist Venen) des Patienten stammen. Die Anzahl der hier zur Verfügung stehenden körpereigenen Gefäßen ist jedoch begrenzt, und bei größeren Operationen oder mehrfachen Wiederholungen von Operationen stehen solche körpereigenen Gefäßen dann nicht mehr zur Verfügung.

Seit langem ist man bemüht, künstliche Arterien als Implantate zu entwickeln und in der Humanmedizin zu verwenden. Bekannt sind dabei Arterien auf Basis von Polyurethan oder von Silicon, wobei insbesondere Silicone aufgrund ihrer ausgezeichneten Bioverträglichkeit bevorzugt werden.

Kleinlumige (Lumen unterhalb 6 mm) künstlichen Gefäße, insbesondre Arterien, müssen im Inneren Endothelzellen aufweisen. Anderenfalls findet innerhalb einer kurzen Zeit ein Verstopfen oder sogar ein Arterienverschluß, der zu dem bekannten, oftmals letalen Konsequenzen führt, statt.

Man hat deshalb bereits versucht, Endothelzellen in das Innere eines künstlichen Blutleiters einzubringen, z.B. durch Pfropfen von Endothelzellen auf die Innenwand eines Blutleiters. Entsprechende Versuche waren jedoch nicht erfolgreich, weil diese Zellen außerordentlich langsam wachsen und auch unter dem Einfluß des strömenden Blutes fortgeschwemmt werden.

Um diesen Nachteil zu überwinden, hat man bereits die Methode der Zellsaat (cell seeding) eingeführt. Bei der Kombination von Kunststoff-Gefäßprothese mit einem autogenen biologischen Material findet eine Vermehrung der Endothelzellen statt. Diese Vermehrung erfolgt dabei so langsam, daß sie eine Thrombosierung nicht unterbinden kann.

Eine Endotheliasierung einer 1 cm langen künstlichen Prothese dauert im besten Fall 6 Wochen. Das bedeutet, daß eine 10 cm lange Prothese 60 Wochen in Anspruch nehmen würde, bis sie funktionsfähig ist. Dies ist jedoch mit dem Leben eines Kranken unvereinbar. Eine Gefäßprothese muß sofort funktionsfähig sein. Das heißt, daß eine künstliche kleinlumige Arterie sofort nach der Implantation schon voll die Funktion der zu ersetzenden Arterie übernehmen muß.

Aufgabe der Erfindung ist es, kleinlumige künstliche Gefäße, insbesondere Arterien, als Implantate zur Verfügung zu stellen, die innerhalb kurzer Zeit über die gesamte Länge nach der Implantation eine ausreichende Epithelialisierung, d.h. Bedeckung der inneren Wandung durch Endothelzellen, aufweist.

Diese Aufgabe wird durch eine kleinlumige Gefäßprothese gelöst, die folgenden Aufbau hat:

A. kleinlumiger mikroporöser Schlauch;

B. Mesh-graft aus autogenem Venenmaterial, das sich enganliegend an der äußeren Oberfläche von A. befindet, und

C. Netz aus einem resorbierbaren Material zum Fixieren von B. auf A.

Das Ausgangsmaterial besteht aus den bekannten künstlichen mikroporösen Schläuchen, insbesondere auf Basis von Polyurethan oder Silicon. Diese Schläuche haben im allgemeinen ein Lumen von 1 bis 3 mm. Das Lumen kann jeweils dem Anwendungszweck, also beispielsweise für die Koronarchirurgie oder die Mikrochirurgie, angepaßt werden.

Die Mikroporosität dieser Schläuche beträgt im allgemeinen 5 bis 500 $\mu$m, vorzugsweise 10 bis 20 $\mu$m.

Dabei ist es vorteilhaft, wenn sich die Porengröße von der Innenseite zur Außenseite des Schlauches vergrößert.

Solche Mikroporen können in bekannter Weise in die künstlichen Schläuche eingebracht werden. Eine Methode besteht beispielsweise darin, daß man den Schlauch aus einer Zusammensetzung aus Polyurethan (PU) und Poly-L-Milchsäure (PLLA) herstellt. Da sich PLLA nach dem Implantieren unter dem Einfluß der Körperflüssigkeiten abbaut, entstehen entsprechende Mikroporen. Eine geeignete Zusammensetzung ist z.B. ein Gemisch aus PU/PLLA von 95/5 Gew.%, wobei die PLLA ein Viskositäts-Durchschnittsmolekulargewicht von etwa 500000 aufweist.

Auf diesen Schlauch wird ein sogenanntes Mesh-graft aufgebracht. Das Mesh-graft besteht aus einem autogenen Material, z.B. einer Vene. Die Vene wird vorher aus dem Körper des bzw. eines Patienten herauspräpariert. Die Vene selbst ist nicht als Arterienersatz geeignet, weil sie ungleiche Konturen hat sowie Ausbuchtungen und Stenosen aufweist. Von dem körpereigenen Gefäß, also beispielsweise der Vene, wird zweckmäßigerweise die äußerste, aus kollagenen und elastischen Fasern aufgebaute Schicht, die Adventitia, und vorzugsweise auch die mittlere Wandschicht, die Media, entfernt. Die in der vorgenannten Weise zweckmäßigerweise präparierte autogene Vene wird durch Einschnitte in Quer- und gegebenenfalls auch in Längsrichtung zu einem Mesh-graft verarbeitet. Ein Mesh-graft im Sinne der vorliegenden Anmeldung ist ein schlauchartiges Gebilde - wie vorher dargelegt wurde, auf Basis eines venösen Materials - und durch die Einschnitte ergeben sich bei einer Längs- und gegebenenfalls Querdehnung eine Maschen(Gitter)-ähnliche Struktur. Da diese Maschenstruktur aus dem autogenen Material auf dem äußeren Umfang des Schlauches aufgebracht ist und in diesen hinein und durch diesen hindurchwächst unter Ausbildung von Endothelzellen im Inneren des Schlauches, wird das ganze Gebilde als Mesh-graft bezeichnet.

Für die Zubereitung des Mesh-graftes aus dem autogenen Material wird die Vene auf ein Plastikröhrchen aufgezogen, wobei der Hohlraum in dem Plastikröhrchen ausreichend groß ist, um den künstlichen Schlauch in diesen Hohlraum einzuführen. Der Hohlraum soll nicht wesentlich größer sein als der für das Implantat verwendete Schlauch; er darf keineswegs kleiner sein, weil sich sonst der Schlauch nicht mehr einführen läßt.

Anschließend wird das Mesh-graft aus dem autogenen Material auf den für das Implantat vorgesehenen Schlauch aufgebracht, indem man es über das Kunststoffröhrchen schiebt, oder indem man von vornherein ein zweigeteiltes Kunststoffrohr verwendet, das dann von beiden Seiten über den darin befindlichen Schlauch und unter dem Meshgraft herausgezogen wird. Als Ergebnis hat man dann die künstliche Gefäßprothese, die mit dem autonomen venösen Mesh-graft bedeckt ist. Dieses Mesh-graft wird auf der künstlichen Gefäßprothese auf die entsprechende Länge gezogen.

Damit das Mesh-graft fest auf dem Kunststoffschlauch, der die Gefäßprothese bildet, sitzt, wird es vorzugsweise in die Poren der Gefäßprothese eingedrückt.

Um eine sichere Haftung des Mesh-grafts auf dem darunter befindlichen Schlauch zu gewährleisten und damit das autogene Material durch die Poren des Schlauches nach innen wächst und dort im Inneren Endothelzellen bildet, ist es erforderlich, das Mesh-graft auf dem künstlichen Gefäß noch weiter zu fixieren. Hierzu eignet sich ein äußeres Netz aus einem geeigneten, biologisch abbaubaren, d.h. resorbierbaren Material. Solche Materialien, die auch als Materialien zur Herstellung resorbierbarer chirurgischer Nähfäden bekannt sind, sind beispielsweise auf Basis von Polyglykolsäure oder Poly-L-Milchsäure aufgebaut. Bei deren Abbau entstehen niedermolekulare Abbauprodukte, die untoxisch und körperverträglich sind. Um das Wachstum der Endothelzellen anzuregen, ist es zweckmäßig, wenn man in dem resorbierbaren Netz Endothelzellen-Wachstumsfaktoren (endothelial cell growth factor [ECGF]) einarbeitet. (Die Bedeutung von ECGF zum stimulieren der Endothelzellen-Proliferation wird von H.P. Greisler et al in Journal of Vascular Surgery, St. Louis, Band 5, Nr. 2, Seiten 393-399, Februar 1987, erläutert.)

Ein Netz aus einem solchen resorbierbaren Kuntstoffmaterial kann auf die zuvor mit dem Mesh-graft präparierte Gefäßprothese aufgebracht werden, indem man die präparierte Gefäßprothese in ein weiteres Plastikröhrchen einschiebt, auf dessen Oberfläche sich ein Netz aus dem resorbierbaren Kunststoffmaterial befindet. Zieht man dann das Plastikröhrchen ab, dann ist die Kunststoffprothese mit dem resorbierbaren Netz bedeckt. Diese Abdeckung mit dem resorbierbaren Netz ist erforderlich, um das Mesh-graft dicht an der Oberfläche der Kunststoffprothese zu halten.

Das erfindungsgemäße Verfahren zur Herstellung einer künstlichen kleinlumigen Gefäßprothese ist dadurch gekennzeichnet, daß man auf einen kleinlumigen mikroporösen Schlauch ein Meshgraft aus autogenem Venenmaterial enganliegend aufbringt, gegebenenfalls das Mesh-graft in den kleinlumigen mikroporösen Schlauch eindrückt und dann ein Netz aus einem resorbierbaren Material auf das Mesh-graft zum Fixieren desselben aufbringt.

Das erfindungsgemäße kleinlumige künstliche Gefäß hat somit vor der Implantation den folgenden Aufbau: Die tragende Substanz im Inneren ist ein Kunststoffschlauch, beispielsweise aus Polyurethan oder Silicon. Auf diesem Kunststoffschlauch befindet sich das Mesh-graft aus dem autogenen Material, wie einer körpereigenen Vene. Über diesem Mesh-graft befindet sich ein Netz aus einem resorbierbaren Polymer.

Ein derart hergestelltes Implantatmaterial zeigt schon kurz nach der Implantation eine schnelle Endothelialisierung im Inneren der Gefäßprothese. Schon nach 3 Tagen kann eine dünne Endothelschicht festgestellt werden. Die Zellen des Meshgrafts Produzieren schon vom ersten Moment der Implantation an Prostaglandine und verhindern damit jegliche Thrombosierung des Blutleiters.

Versuchsbericht:

Für den Versuch wurden Wistar-Ratten mit einem Gewicht zwischen 280 und 330 g verwendet. Die Ratten wurden mit einer Standard-Rattennahrung und mit ausreichend Wasser ernährt.

Als Prothesematerial wurde mikroporöses Polyurethan in Form eines Schlauches verwendet mit einem Innendurchmesser von 1,5 mm und einer Wanddicke von 0,3 mm. Die mittlere Porengröße beträgt 30 $\mu$m an der Innenseite und 100 $\mu$m an der Außenseite.

Die Ratten wurden zunächst mit Aropin vorbehandelt (0,25 mg/1 kg Körpergewicht, i.m.), mit 1 Volumen-% Halotane und mit einer 2 : 1-Mischung aus $N_2O$ und Sauerstoff anästesiert. Unter sterilen Bedingungen wurde ein 5 mm langes Segment aus der rechten Arteria carotis entfernt. Mit einer mikrochirurgischen Schere wurde in dem resezierten Segment quer zur Länge kleine Einschnitte vorgenommen, wodurch sich ein Mesh bildete, das von 5 mm auf 10 mm Länge vergrößert war. Anschließend wurden in dem Mesh Längsschnitte vorgenommen. Dieses netzförmige Material wurde auf das mikroporöse Polyurethan-Prothesenmaterial aufgezogen und mit einem Netz aus einem resorbierbaren Material auf Basis von Polymilchsäure dort fixiert. Dann wurde ein Einschnitt im Abdominalbereich vorgenommen und die abdominale Aorta freigelegt. Nach der Ligatur der beiden iliolumbalen Äste wurde ein 20 mm-Infrarenalesegment der abdominalen Aorta sorgfältig von anliegendem vena cava und umgebendem Gewebe befreit. Ein 12 mm-Segment der Aorta wurde dann mittels zweier mikrochirurgischer Klammern abgeklemmt und 10 mm der Aorta wurden reseziert. Die Gefäßenden wurden mit einer Lösung, enthaltend 1 Einheit Heparinnatrium, gespült. Anschließend daran wurde die vorher hergestellte mikroporöse Vaskularprothese implantiert. Es wurde eine End-zu-End-Anastomose mit einem resorbierbaren Nahtmaterial, Ethilon[R]9-0 (BV-4-Nadel von Ethicon Inc., Sommerville, N.Y., USA) durchgeführt. Anschließend versicherte man sich, daß der arterielle Puls wieder vorhanden war. Die Operationsstelle wurde dann mit einer Kochsalzlösung gespült und mit Dexon[R]4-0-Nahtmaterial geschlossen. Die Tiere erhielten keine antithrombotischen Mittel.

Die Implantate wurden am 8., 16., 24. und 135. Tag nach der Implantation untersucht. Vor der Entfernung der Implantate wurde Heparin (1000 IU i.v.) verabreicht, um einen Verschluß zu verhindern. Die Durchgängigkeit wurde makroskopisch bewertet. Die Abdominalaorta wurde proximal und distal von dem Prothesematerial abgeklammert. Unmittelbar nach dem Abklammern wurde das Prothesematerial entnommen und mit 0,9% Natriumchlorid und anschließend mit 2% Glutaraldehyd in 0,1 M Phosphatpuffer, pH-Wert 7,4, während einer Minute gespült. Die Proben wurden entweder 24 Stunde in 2% Glutaraldehyd gelagert und dann einer Elektronenabtast-Mikroskopie unterworfen oder für eine Untersuchung mit dem Lichtmikroskop in 4% Formaldehyd gelagert.

Zubereitung für die Lichtmikroskopie:

Die Proben wurden längs oder kreisförmig geschnitten. Sie wurden fixiert und dann mit Ethanol dehydratisiert. Anschließend erfolgte eine Dehydrationseinbettung in Glykolmethacrylat gemäß Blaauw, E.H., Jonkman, M.F. and Gerrits, P.O.: A rapid connective tissue stain for glycol methacrylate embedded tissue. Acta Morphol. Neerl.-Scand. 25 (1987)167-172. Im Anschluß daran wurde mit Toluidinblau und alkalischem Fuchsin gefärbt.

Zubereitung für die Elektronenabtast-Mikroskopie:

Die Gewebeproben wurden 30 Minuten mit einer 6,8%-igen Saccharoselösung mit Phosphatpuffer auf pH 7,4 gepuffert, gespült und dann 3 Stunden mit 1% Osmiumtetroxid in 0,1% Phosphatpuffer, pH 7,4 bei 4°C fixiert. Nach dem Dehydratisieren in Alkohol und Spülen mit Amylacetat wurden die Proben mit Kohlendioxid gefriergetrocknet. Im Anschluß daran wurden die Proben mit Gold-Palladium sputterbeschichtet. Die Segmente wurden mit einem JSM 35 C-scanning-Elektronenmikroskop, das bei 15 bis 25 kV betrieben wurde, untersucht.

Ergebnisse:

Makroskopische Untersuchung:

Alle untersuchten Probestücke waren durchlässig. Eine Pulsierung an der distalen Aorta wurde festgestellt.

Aneurismatische oder stenotische Veränderungen der Aorta wurden nicht festgestellt. Das vaskulare Mesh-graft war vital und eng an dem mikroporösen Schlauch befestigt.

Lichtmikroskopie:

In allen Proben wurde eine Migration der Zellen von dem Mesh-graft in die luminale Oberfläche des Prothesematerials festgestellt. Die Poren der Wandung waren mit Zellmaterial gefüllt.

Das Lumen der endothelialisierten Schläuche war mit 5 bis 10 Schichten Zellmaterial bedeckt. Es konnte keine Nekrosis des vaskularen Mesh-grafts festgestellt werden.

Elektronenabtast-Mikroskopie:

Bei der Probe, die nach 8-tägiger Implantation erhalten wurde, wurden Zellen festgestellt, die durch die Poren in das Lumen eingewachsen waren und sich über die gesamte innere Oberfläche erstreckten. Große Teile des Lumens waren vollständig mit Endothelzellen bedeckt, während andere noch nicht vollständig endothelialisiert waren und Leukozyten und Thrombozyten mit Fibrin enthielten.

**Ansprüche**

1. Künstliche kleinlumige Gefäßprothese, **gekennzeichnet** durch folgenden Aufbau (von innen nach außen):

A. kleinlumiger mikroporöser Schlauch;

B. Mesh-graft aus autogenem Venenmaterial, das sich enganliegend an der äußeren Oberfläche von A. befindet, und

C. Netz aus einem resorbierbaren Material zum Fixieren von B. auf A.

2. Künstliche Gefäßprothese gemäß Anspruch 1, dadurch **gekennzeichnet**, daß A. ein Schlauch auf Basis von Polyurethan oder Silicon ist.

3. Gefäßprothese gemäß Anspruch 1, dadurch **gekennzeichnet,** daß C. aus einem resorbierbaren Material auf Basis von Polyglykolsäure oder Poly-L-Milchsäure ist.

4. Mesh-graft gemäß Anspruch 1, dadurch **gekennzeichnet,** daß A. Mikroporen mit einer mittleren Porengröße zwischen 5 und 500 μm aufweist.

5. Gefäßprothese gemäß Anspruch 4, dadurch **gekennzeichnet,** daß die mittlere Porengröße 10 bis 200 μm beträgt.

6. Gefäßprothese gemäß Anspruch 5, dadurch **gekennzeichnet,** daß die Porengröße von der Innenseite zur Außenseite zunimmt.

7. Verfahren zur Herstellung von künstlichen kleinlumigen Gefäßprothesen, dadurch **gekennzeichnet,** daß man auf einen kleinlumigen mikroporösen Schlauch ein Mesh-graft aus autogenem Venenmaterial enganliegend aufbringt, gegebenenfalls das Mesh-graft in den kleinlumigen mikroporösen Schlauch eindrückt und dann ein Netz aus einem resorbierbaren Material auf das Mesh-graft zum Fixieren desselben aufbringt.

Europäisches
Patentamt

# EUROPÄISCHER RECHERCHENBERICHT

Nummer der Anmeldung

EP 89 10 8584

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 308 102  (UBE IND. LTD)<br>* Ansprüche 1-3,8,9; Figur 1 *<br>--- | 1-5 | A 61 F   2/06 |
| Y | EP-A-0 194 192  (ETHNOR)<br>* Zusammenfassung; Seite 6, Zeilen 1-8;<br>Seite 7, Zeilen 22-31; Ansprüche<br>1-3,11,14,15 *<br>--- | 1-5 | |
| A | US-A-4 208 745  (K. OKITA)<br>* Zusammenfassung *<br>--- | 6 | |
| A | WO-A-8 203 764  (MASSACHUSETTS<br>INSTITUTE OF TECHNOLOGY)<br>----- | | |

**RECHERCHIERTE SACHGEBIETE (Int. Cl.5)**

A 61 F

Der vorliegende Recherchenbericht wurde für alle Patentansprüche erstellt

| Recherchenort | Abschlußdatum der Recherche | Prüfer |
|---|---|---|
| DEN HAAG | 18-12-1989 | SANCHEZ Y SANCHEZ J. |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes
   Dokument

EPO FORM 1503 03.82 (P0403)